Europäisches Patentamt

**(19)** European Patent Office

Office européen des brevets

**(11)** Publication number : **0 313 282 B1**

**(12)** EUROPEAN PATENT SPECIFICATION

**(45)** Date of publication of patent specification :
01.04.92 Bulletin 92/14

**(51)** Int. Cl.⁵ : **C01B 13/32,** C01B 9/00,
C01G 1/06

**(21)** Application number : 88309669.5

**(22)** Date of filing : 14.10.88

**(54)** Oxidation of metal (oxo)halides.

**(30)** Priority : 20.10.87 GB 8724497

**(43)** Date of publication of application :
26.04.89 Bulletin 89/17

**(45)** Publication of the grant of the patent :
01.04.92 Bulletin 92/14

**(84)** Designated Contracting States :
AT BE CH DE ES FR GB IT LI NL

**(56)** References cited :
GB-A- 1 397 742
GB-B- 1 462 049
GB-B- 1 462 050

**(73)** Proprietor : **The British Petroleum Company
p.l.c.
Britannic House Moor Lane
London EC2Y 9BU (GB)**

**(72)** Inventor : **Seddon, Kenneth Richard
Upper St. Mary's Mead Broadhill Ockley Lane
Keymar West Sussex BN6 8PA (GB)**

**(74)** Representative : **Krishnan, Suryanarayana
Kalyana et al
BP INTERNATIONAL LIMITED Patents &
Agreements Division Chertsey Road
Sunbury-on-Thames Middlesex TW16 7LN
(GB)**

## Description

The present invention relates to a process for oxidizing metal (oxo)halides to the corresponding simple or complex metal oxohalides or metal oxides.

It is well known to produce metal oxides by dehydration of the corresponding metal hydroxides, by simple oxidation of metals in air or dioxygen, or by decomposition of metal carbonates. For producing complex metal oxides, the respective oxides of the metals have been fused together. All these processes involve the use of relatively high temperatures, in many cases above 500°C. For instance, to produce lithium niobate(V) by fusion of the respective oxides, temperatures well in excess of 500°C are required. Similarly, production of lithium oxide by dehydration of the corresponding hydroxide requires temperatures in excess of 200°C.

Such relatively high temperature reactions are unsuitable for some purposes, especially if the oxides have to be deposited on or within a temperature sensitive substrate, or, if such oxides are to be produced from non-oxygen containing salts such as the halides.

It has now been found that such metal oxides and oxohalides, whether simple or complex, can be produced from the corresponding salts at relatively low temperatures, e.g. ambient temperature, if they are reacted with the appropriate oxidizing agents.

Accordingly, the present invention is a process for producing simple or complex metal oxide(s) or oxohalide(s), said process comprising oxidizing an oxohalide and/or a halide of one or more metals using an iodoso aromatic compound as the oxidizing agent.

The present oxidation reaction will proceed with most metal (oxo)halides, although its effectiveness is probably greater the higher the formal oxidation state of the product oxide or oxohalide. Thus, the (oxo)halide of a metal such as niobium or vanadium is more readily oxidized than e.g. an alkali metal (oxo)halide. The oxidation process of the present invention is particularly suited for producing simple oxides and oxohalides of the transition metals such as e.g. vanadium, niobium, chromium, titanium, iron and the like, and for producing binary and ternary oxides and oxohalides such as e.g. $LiNbO_3$, $BaTiO_3$, $LiVO_3$ and the like.

The oxidizing agent used is an aromatic iodoso compound represented by the formula ArIO wherein 'Ar' represents one or more aromatic nuclei. Examples of aromatic nuclei that may be used include phenyl, naphthyl, anthracyl and substituted derivatives thereof. The substituents in such nuclei may be non-functional such as alkyl groups or functional groups such as e.g. hydroxyl, halogen or carboxyl. The simplest iodosoaromatic compound is iodosobenzene, $C_6H_5IO$, hereafter referred to as "PhIO", whereas typical representatives of the substituted iodoso aromatic compound are a dimethyl iodosobenzene such as 2,5-dimethyliodosobenzene, and a fluoro substituted derivative, e.g. pentafluoroiodosobenzene, $C_6F_5IO$.

The amount of iodosoaromatic compound used with respect to the metal (oxo)halide to be oxidised will depend upon the nature of the starting metal halide or metal oxohalide, the oxidizing agent chosen, and the reaction conditions. It will also depend upon whether the metal halide is to be oxidized to an oxohalide or through to the metal oxide, or whether the end product is a complex metal oxide such as e.g. the ternary oxide lithium niobate(V).

The oxidation reaction may be carried out in the homogeneous or heterogeneous phase, although it is preferable to carry out the oxidation under homogeneous conditions.

Thus, the reaction is preferably carried out in the presence of a solvent. The solvent, if any, used should be such that it does not compete with the metal (oxo)halide in the oxidation reaction. It is also preferable to use oxidizing conditions which avoid the formation of hydroxides from the metal (oxo)halide. This is due to the fact that the conversion of metal hydroxides to the corresponding oxides can require relatively more severe conditions than if such hydroxide formation is avoided. Thus, the presence of water and alcoholic solvents may favour the conversion of the metal (oxo)halides into hydroxides. Hence, it is preferable to carry out the oxidation under anhydrous conditions and in the presence of readily volatile solvents such as e.g. ethanenitrile, nitromethane, tetrahydrofuran and the like.

In order to facilitate the solubilization of the starting materials, the metal (oxo)halides may be complexed prior to contact with the oxidizing agent. Typical examples of such solubilized complexes include the pyridinium salt and the tetraalkylammonium salt of the metal (oxo)halide. Thus, pyridinium hexachlorochromate(III) and pyridinium hexachlorovanadate(III) readily dissolve in ethanenitrile. Such pyridinium salts also have the advantage in some cases of enabling the oxidized product to separate out of the reaction mixture as a solid. Thus, for instance, pyridinium hexachlorovanadate(III) upon oxidation with iodosobenzene in ethanenitrile at room temperature allows solid pyridinium oxotetrachlorovanadate(V) to separate out as a solid from the reaction mixture in solution. Similarly, the tetramethylammonium hexachloroniobate(V) also dissolves readily in nitromethane.

The oxidizing reaction can in most cases proceed in a single step at ambient temperature, thus avoiding the use of expensive or complicated equipment either for the reaction or for the work-up of the products.

When the reaction is completed, the iodoso aromatic oxidizing agent itself is transformed into a halo-aromatic compound which is readily removed from the metal oxide or oxohalide. PhIO thus gives rise to iodobenzene when reduced. As this iodobenzene may be recovered and reoxidized to PhIO, this may form the basis of a catalytic cycle. In the case of chlorocompounds being used as reactants, chlorine has been identified as the other inorganic oxidation product.

The relatively mild reaction conditions enable the method to be used, for instance, for producing (a) oxides with labelled oxygen atoms e.g. $^{17}O$ which are suitable for medical uses, (b) polymer matrices with oxides deposited in-situ which can be used in optical devices, (c) temperature sensitive, oxide containing electronic and semi-conductor devices, and (d) simple and complex metal oxides and oxohalides at very low temperatures thereby enabling considerable savings in costs relating to equipment and energy.

The present invention is further illustrated with reference to the following Examples.

Example 1

Niobium(V) chloride (0.5g) was dissolved in ethanenitrile (10 cm$^3$) and added to a suspension of iodosobenzene (1.2g) in ethanenitrile (10 cm$^3$). The mixture was heated at reflux for 2.5h, to produce a quantitative white precipitate of niobium(V) oxide.

Example 2

Niobium(V) chloride (0.5g) was dissolved in nitromethane (10 cm$^3$) and added to a suspension of iodosobenzene (1.2g) in nitromethane (10 cm$^3$). The mixture was heated at reflux for 2.5h, to produce a quantitative white precipitate of niobium(V) oxide.

Example 3

Niobium(V) chloride (0.5g) was dissolved in tetrahydrofuran (10 cm$^3$) and added to a suspension of iodosobenzene (1.2g) in tetrahydrofuran (10 cm$^3$). The mixture was heated at reflux for 2.5h, to produce a quantitative white precipitate of niobium(V) oxide.

Example 4

Niobium(V) chloride (0.5g) was dissolved in ethanenitrile (10 cm$^3$) and added to a suspension of iodosobenzene (1.2g) in ethanenitrile (10 cm$^3$) in the presence of lithium chloride (0.08g). The mixture was heated at reflux for 2.5h, to produce a white precipitate of lithium niobate(V).

Example 5

Iron(III) chloride (0.05g) was dissolved in ethanenitrile (10 cm$^3$) and added to a suspension of iodosobenzene (0.2g) in ethanenitrile (10 cm$^3$). The mixture was heated at reflux for 2.5h, to produce a quantitative brown precipitate of iron(III) oxide.

Example 6

Pyridinium hexachlorochromate(III) (0.1g) was dissolved in ethanenitrile (5 cm$^3$) and to this was added solid iodosobenzene (1g). The mixture was stirred at room temperature for 5 minutes, and then quenched in water (40 cm$^3$) to produce a solution containing chromate(VI) ions, produced quantitatively from the chromium(III) complex.

Example 7

Pyridinium hexachlorovanadate(III) (0.1g) was dissolved in ethanenitrile (5 cm$^3$) and to this was added solid iodosobenzene (1g). The mixture was stirred at room temperature for 5 minutes, and then quenched in water (40 cm$^3$) to produce solid vanadium(V) oxide, produced quantitatively from the vanadium(III) complex. The solution, prior to quenching, contained pyridinium vanadate(V).

Example 8

Pyridinium hexachlorovanadate(III) (0.3g) was dissolved in ethanenitrile (5 cm$^3$) and to this was added solid iodosobenzene (0.15g). The mixture was stirred at room temperature for 5 minutes, whence pyridinium oxotetrachlorovanadate(V) formed and separated as a solid.

Example 9

Titanium(IV) chloride (0.05g) was dissolved in ethanenitrile (10 cm$^3$) and added to a suspension of iodosobenzene (0.2g) in ethanenitrile (10 cm$^3$). The mixture was heated at reflux for 2.5h, to produce a quantitative white precipitate of titanium(IV) oxide.

Example 10

Niobium(V) chloride (0.15g) was dissolved in ethanenitrile (10 cm$^3$) and added to a suspension of pentafluoroiodosobenzene (0.47g) in ethanenitrile (10 cm$^3$). The mixture was heated at reflux for 2.5 hours to produce a quantitative white precipitate of niobium(V) oxide.

Example 11

Niobium(V) chloride (0.20g) was dissolved in nitromethane (10 cm$^3$) and added to a suspension of pentafluoroiodosobenzene (0.5g) in nitromethane (10 cm$^3$). The mixture was heated at reflux for 2.5 hours to produce a quantitative white precipitate of niobium(V)oxide.

## Example 12

Tetraethylammonium hexachloroniobate(V) (0.18g) was dissolved in nitromethane (10 cm³) and added to a suspension of pentafluoroiodosobenzene (1.0g) in nitromethane (10 cm³). The mixture was heated at reflux for 1.5 hours to produce a quantitative white precipitate of niobium(V)oxide.

## Example 13

Iron(III) chloride (0.15g) was dissolved in ethanenitrile (10 cm³) and added to a suspension of pentafluoroiodosobenzene (0.47g) in ethanenitrile (10 cm³). The mixture was heated at reflux for 2 hours to produce a quantitative brown precipitate of iron(III)oxide.

## Example 14

Iron(III) chloride (0.20g) was dissolved in nitromethane (10 cm³) and added to a suspension of pentafluoroiodosobenzene (1.0g) in nitromethane (10 cm³). The mixture was heated at reflux for 2 hours to produce a quantitative brown precipitate of iron(III)oxide.

## Example 15

Niobium(V) chloride (0.5g) was dissolved in ethanenitrile (10 cm³) and added to a suspension of pentafluoroiodosobenzene (1.4g) in ethanenitrile (10 cm³) in the presence of lithium chloride (0.08g). The mixture was heated at reflux for 2.5 hours to produce a white precipitate of lithium niobate(V).

## Example 16

Titanium(IV) chloride (0. 10g) was dissolved in ethanenitrile (10 cm³) and added to a suspension of pentafluoroiodosobenzene (0.40g) in ethanenitrile (10 cm³). The mixture was heated at reflux for 1.5 hours to produce a quantitative white precipitate of titanium(IV)oxide.

## Example 17

Pyridinium hexachlorovanadate(III) (0.1g) was dissolved in ethanenitrile (5 cm³) and to this was added solid pentafluoroiodosobenzene (1.2g). The mixture was stirred at room temperature for 5 minutes and then quenched in water (40 cm³) to produce solid vanadium(V)oxide, produced quantitatively from the vanadium(III)complex. The solution, prior to quenching, contained pyridinium vanadate(V).

## Examples 18 and 19 (using 2,5-dimethyliodosobenzene)

### A. Synthesis of 2,5-dimethyliodosobenzene

(a) Synthesis of 2,5-dimethylphenyliodine(III) dichloride.

2,5-dimethyliodobenzene (1.0 g), dissolved in trichloromethane (5 cm³), was chlorinated by the passage of dichlorine (10 cm³ min⁻¹ for 1 h) over the surface of the stirred solution at -10°C in the dark. The yellow solid was collected by filtration, dissolved in trichloromethane (5 cm³; -10°C), and precipitated by the addition of petroleum ether (60-80; -10°C). The mixture was allowed to stand at -10°C for 5 h, and the solid was then collected by filtration, washed with a trichloromethane/petroleum ether (60-80) mixture (80:20; -10°C), and dried. Yield: 0.9 g (69%). ¹H n.m.r. (CDCl₃; -10°C) /p.p.m. 8.01 (S, 1H; 6-H), 7.32 (dd, 2H, $J_{3,4}$ = 7.8 Hz; 3,4-H₂), 2.77 (s, 3H; 5-CH₃), 2.35 (s, 3H; 2-CH₃).

(b) Synthesis of 2,5-dimethyliodosobenzene

Anhydrous sodium carbonate (2 g) and crushed ice (3 g) were added to 2,5-dimethylphenyliodine(III) dichloride (5.5 g) contained in a large ice-chilled mortar. The mixture was then ground to a thick paste, and aqueous sodium hydroxide solution (5 M, 3 cm³) was added in six equal aliquots, with repeated trituration between each addition. The mixture was then diluted with water (10 cm³) and allowed to stand overnight at room temperature. The yellow solid product was collected by filtration, washed repeatedly with water (6 x 10 cm³), and allowed to dry in the air on filter paper. The dry product was then suspended in trichloromethane (2 cm³), collected by filtration, and again air-dried. Yield: 4.2 g (93%). Found; C, 38.3; H, 3.3%. Calc. for C₈H₉IO: C, 38.7; H, 3.65%.

### B. Use of 2,5-dimethyliodosobenzene

### Example 18

Pyridinium hexachlorovanadate(III) (0.50 g) was dissolved in ethanenitrile (20 cm³) under a dinitrogen atmosphere to give a purple solution. 2,5-Dimethyliodosobenzene (0.246 g) was then added, and the mixture was stirred vigorously. The colour changed from purple to green within one minute. The mixture was filtered to yield a mixture of green and blue solids, which were the two isomers of pyridinium oxotetrachlorovanadate(IV); [C₅H₆N]₂[VOCl₄]. These isomers were separated by fractional recrystallisation. Found for blue isomer: C, 32.3; H, 4.2; N, 7.8%. Calc. for C₁₀H₁₂Cl₄N₂OV: C, 32.6; H, 3.3; N, 7.6%. Found for green isomer: C, 32.6; H, 4.1; N, 7.7%.

Calc. for $C_{10}H_{12}Cl_4N_2OV$: C, 32.6; H, 3.3; N, 7.6%.

Example 19

Pyridinium hexachlorovanadate(III) (0.50 g) was dissolved in ethanenitrile (20 cm³) under a dinitrogen atmosphere to give a purple solution. 2,5-Dimethyliodosobenzene (0.494 g) was then added, and the mixture was stirred vigorously. The colour changed from purple to green within one minute. The mixture was filtered to yield a mixture of green and blue solids, which where the two isomers of pyridinium oxotetrachlorovanadate(IV). These isomers were separated by fractional recrystallisation, and were identical to those prepared in Example 18.

This facility to produce metal oxides at low temperatures enables such oxides to be deposited on temperature sensitive substrates eg. as protective coatings. The metal oxohalides formed can also act as oxidising agents.

**Claims**

1. A process for producing simple or complex metal oxide(s) or oxohalide(s), said process comprising oxidizing an oxohalide and/or a halide of one or more metals with an iodosoaromatic compound.

2. A process according to claim 1 wherein the iodosoaromatic compound has the formula ArIO wherein Ar represents one or more aromatic nuclei or substituted derivatives thereof.

3. A process according to claim 2 wherein the substituents for the aromatic nuclei are selected from non-functional and functional groups.

4. A process according to any one of the preceding claims wherein the iodosoaromatic compound is selected from iodosobenzene, dimethyliodosobenzene and pentafluoroiodosobenzene.

5. A process according to any one of the preceding claims wherein the oxidation is carried out in a solvent under homogeneous conditions.

6. A process according to claim 5 wherein the solvent is anhydrous.

7. A process according to any one of the preceding claims wherein the metal halide and/or oxohalide is complexed prior to contact with the iodosoaromatic compound.

8. A process according to claim 7 wherein the metal halide and/or oxohalide is complexed as its pyridinium salt or as its tetraalkyl ammonium salt.

9. A process according to any one of the preceding claims wherein the metal halide(s) and/or oxohalide(s) oxidised are of transition metals.

10. A process according to claim 9 wherein metal halide or oxohalide which is oxidised is of a transition metal selected from those of vanadium, niobium, chromium, titanium and iron.

**Patentansprüche**

1. Verfahren zur Herstellung von einem einfachen oder komplexen Metalloxid oder -oxohalogenid (von einfachen oder komplexen Metalloxiden oder -oxohalogeniden), welches Verfahren das Oxidieren von einem Oxohalogenid und/oder einem Halogenid von einem oder mehreren Metallen mit einer aromatischen Iodosoverbing umfaßt.

2. Verfahren nach Anspruch 1, worin die aromatische Iodosoverbindung die Formel ArIO besitzt, worin Ar einen oder mehrere aromatische Kerne oder substituierte Derivate hievon darstellt.

3. Verfahren nach Anspruch 2, worin die Substituenten für die Aromatischen Kerne unter nicht-funktionellen und funktionellen Gruppen ausgewählt sind.

4. Verfahren nach einem der vorstehenden Ansprüche, worin die aromatische Iodosoverbindung unter Iodosobenzol, Dimethyliodosobenzol und Pentafluoriodosobenzol ausgewählt ist.

5. Verfahren nach einem der vorstehenden Ansprüche, worin die Oxidation in einem Lösungsmittel unter homogenen Bedingungen ausgeführt wird.

6. Verfahren nach Anspruch 5, worin das Lösungsmittel wasserfrei ist

7. Verfahren nach einem der vorstehenden Ansprüche, worin das Metallhalogenid und/oder das -oxohalogenid komplexiert wird, bevor es mit der aromatischen Iodosoverbindung in Kontakt gebracht wird.

8. Verfahren nach Anspruch 7, worin das Metallhalogenid und/oder das -oxohalogenid als sein Pyridiniumsalz oder als sein Tetraalkylammoniumsalz komplexiert wird.

9. Verfahren nach einem der vorstehenden Ansprüche, worin das oxidierte Metallhalogenid und/oder -oxohalogenid (die oxidierten Metallhalogenide ynd/oder -oxohalogenide) von Übergangsmetallen stammt (stammen).

10. Verfahren nach Anspruch 9, worin das Metallhalogenid oder -oxohalogenid, welches oxidiert wird, eines von einem Übergangsmetall ist, welches unter Vanadium, Niob, Chrom, Titan und Eisen ausgewählt ist.

**Revendications**

1. Procédé de production d'oxyde(s) ou d'oxyhalogénure(s) métalliques simples ou complexes, ledit procédé comprenant l'oxydation de l'oxyhalogénure et/ou d'un halogénure d'un ou plusieurs métaux avec un composé iodoso-aromatique.

2. Procédé suivant la revendication 1, dans lequel le composé iodoso-aromatique répond à la formule ArIO dans laquelle Ar représente un ou plusieurs noyaux aromatiques ou leurs dérivés substitués.

3. Procédé suivant la revendication 2, dans lequel les substituants des noyaux aromatiques sont choisis entre des groupes non fonctionnels et des groupes fonctionnels.

4. Procédé suivant l'une quelconque des revendications précédentes, dans lequel le composé iodosoaromatique est choisi entre l'iodosobenzène, le diméthyliodosobenzène et le pentafluoriodosobenzène.

5. Procédé suivant l'une quelconque des revendications précédentes, dans lequel l'oxydation est conduite dans un solvant dans des conditions homogènes.

6. Procédé suivant la revendication 5, dans lequel le solvant est anhydre.

7. Procédé suivant l'une quelconque des revendications précédentes, dans lequel l'halogénure et/ou l'oxyhalogénure métalliques sont complexés avant leur mise en contact avec le composé iodoso-aromatique.

8. Procédé suivant la revendication 7, dans lequel l'halogénure et/ou l'oxyhalogénure métallique sont complexés sous forme de leur sel de pyridinium ou de leur sel de tétraalkylammonium.

9. Procédé suivant l'une quelconque des revendications précédentes, dans lequel l'halogénure ou les halogénures et/ou l'oxyhalogénure ou les oxyhalogénures métalliques oxydés sont des composés de métaux de transition.

10. Procédé suivant la revendication 9, dans lequel l'halogénure ou l'oxyhalogénure métallique qui est oxydé est un composé de métal de transition choisi parmi les composés correspondants de vanadium, de niobium, de chrome, de titane et de fer.